# EUROPEAN PATENT SPECIFICATION

(11) **EP 0 668 748 B1**
(45) Date of publication and mention of the grant of the patent: **19.01.2000**
(21) Application number: 92925137.9
(22) Date of filing: 13.11.1992
(51) Int. Cl.: A61B 17/08

(54) **APPARATUS FOR THE CLOSURE OF WIDE SKIN DEFECTS BY STRETCHING OF SKIN**
VORRICHTUNG ZUM VERSCHLIESSEN BREITER HAUTWUNDEN DURCH HAUTSPANNUNG
APPAREIL DE FERMETURE DE PLAIES LARGES PAR ETIRAGE DE LA PEAU

(43) Date of publication of application: 30.08.1995
(73) Proprietor: MEDCHEM PRODUCTS, INC., Princeton, NJ 08540 (US)
(72) Inventor: HIRSHOWITZ, Bernard, 34 464 Haifa (IL); LEVY, Amnon, 34 323 Haifa (IL)
(74) Representative: Kador & Partner
(86) International application number: US9209669
(87) International publication number: WO9410916

(56) References cited:
- EP-A- 0 018 698
- EP-A- 0 269 935
- EP-A- 0 279 534
- US-A- 2 450 194
- US-A- 3 971 384
- US-A- 4 747 394

## Description

### BACKGROUND OF THE INVENTION

The invention relates to apparatus serving to stretch skin so as to cover an open wound and to enable the surgeon to suture together the opposite skin margins. It relates especially to apparatus to be used during operations following wide excision of skin lesions or for closure of skin defects or otherwise damaged skin areas, thereby obviating the conventional method of using local skin flaps or grafting of skin from other body portions.

U.S. Patent No.4,896,680, by one of the present inventors, Bernard Hirshowitz, describes a method and apparatus for stretching the skin over a wound by load cycling including applying a pulling force on opposite skin margins during several periods interrupted by relaxation periods. In this way the skin can be stretched over a wide area in a manner not previously used until the invention by Bernard Hirshowitz. The above patent discloses a surgical stretching apparatus which comprises two pins to be inserted into the skin along both edges of the wound which are gradually pulled together by means of a flexible strap. According to the invention the pulling is done in intervals to allow the collagen fibers of the skin to rearrange themselves for further stretching. The pins of the described apparatus are more or less in the shape of safety pins each provided with a loop for attachment to the flexible strap; the strap, for its part, has projections or apertures for engagement with a ratchet-shaped device which will hold the two pins in forceful apposition.

It has, however, been shown that the described apparatus has certain drawbacks which should be overcome by the present invention, the main drawback being that after the two pins have been drawn together, they do not leave room for suturing the approximated akin margins. Another drawback is that manual pulling on the flexible strap is rather crude, in that the pulling force cannot be minutely controlled. And finally that the two pins grip only relatively narrow strips of skin along both edges, thereby not permitting closure of a wide wound by one pulling operation.

It is, therefore, an object of the invention to provide apparatus which will permit suturing together of the skin edges while the stretching apparatus is in situ without disturbing the suturing operation.

It is another object to permit insertion into the skin edges of longer pins adapted to grip practically the entire length of the wound and to permit shifting of the stretching means along the wound edges thereby enabling the two pins to be engaged in different locations.

And it is a further object to permit prolonged use of the apparatus as well as of the pins, contrariwise to the apparatus described in the previous patent which had to be disposed after one use.

### SUMMARY OF THE INVENTION

There is accordingly provided an apparatus for closing of skin defects as defined in claim 1.

A preferred embodiment of the apparatus comprises essentially; 1. two long pins to be inserted under the skin along the two edges of a wound to be closed; 2. two U-shaped retaining members each having a top surface, and a bottom surface to be placed close to the skin, each member being provided with a sharp hook at the end of each leg of the "U"; 3. Contracting means for pulling and approximating the two retaining members with their legs extending towards the open wound. The retaining members are adapted to be placed behind the pins in opposite alignment with their hooks piercing the skin and engaging the respective pin in a position along the wound edges. The contracting means are designed to pull the two retaining members slowly and gradually together so as not to cause damage to the skin area. The U-shape permits suturing of the skin edges with the apparatus in position, due to the open spaces left between and on both sides of the two legs.

A preferred embodiment of the contracting means comprises two contractor arms in parallel alignment, connected by a screw means adapted to approximate the two components by rotating the screw means. One end of each contractor arm is in the shape of a bar of smooth, even cross section adapted to retain one retaining member each in opposite alignment on both sides of a wound and to allow shifting of each retaining member along its respective bar. The contractor arms are preferably slidably connected at their other ends by a smooth bar engaging with bores in corresponding locations with a view to keeping the two arms in parallel alignment. Each U-shaped retaining member comprises two legs with outwardly bent hooks at the bottom surface of their ends connected by a longitudinally perforated flange adapted to be singly mounted on the smooth bar of a contractor arm.

After the two pins have been inserted along the wound margins, the apparatus is placed across the wound with the retaining members positioned behind the skin margins, while the hooks are pressed through the skin into engagement with the pins. Now the screw means is rotated so as to slowly approximate the two contractor arms and the attached retaining members and the operation is continued at intervals as described in the aforementioned patent.

Another embodiment of the apparatus comprises two U-shaped retaining members having forwardly bent hooks attached to the underside of the legs at their respective ends, a connecting flange provided with a lug protruding out of its top surface which is perforated and provided with screw thread for engagement with a screw adapted to be rotated for the purpose of approximating the two members. A preferred embodiment of the apparatus includes one screw-threaded member and one smooth-bored member, and a screw provided with a collar at its one end engaging with the smooth bore and screw thread over its remaining length engaging with the screw-threaded member. The drawback of this apparatus, compared with the afore-described one, lies in the obstruction caused by the screw preventing suturing of the area between the two legs.

Other contracting means may be provided for approximating the two retaining members, but it will be understood that the essence of the invention lies in the shape of the two retaining members which permits suturing of the wound margins while the apparatus is still in situ, pulling the margins together.

### BRIEF DESCRIPTION OF THE DRAWINGS.

**Figure 1** is a plan view of an apparatus positioned over an open skin wound,
**Figure 2** is a section through the apparatus along line 2-2 of Figure 1,
**Figure 3** is a section through the apparatus along line 3-3 of Figure 1,
**Figure 4** is a side view of the apparatus along line 4-4 of Figure 1,
**Figure 5** is a top view of another embodiment of the apparatus of the invention, and
**Figure 6** is a side view of the apparatus illustrated in Figure 5.

### DETAILED DESCRIPTION OF THE DRAWINGS

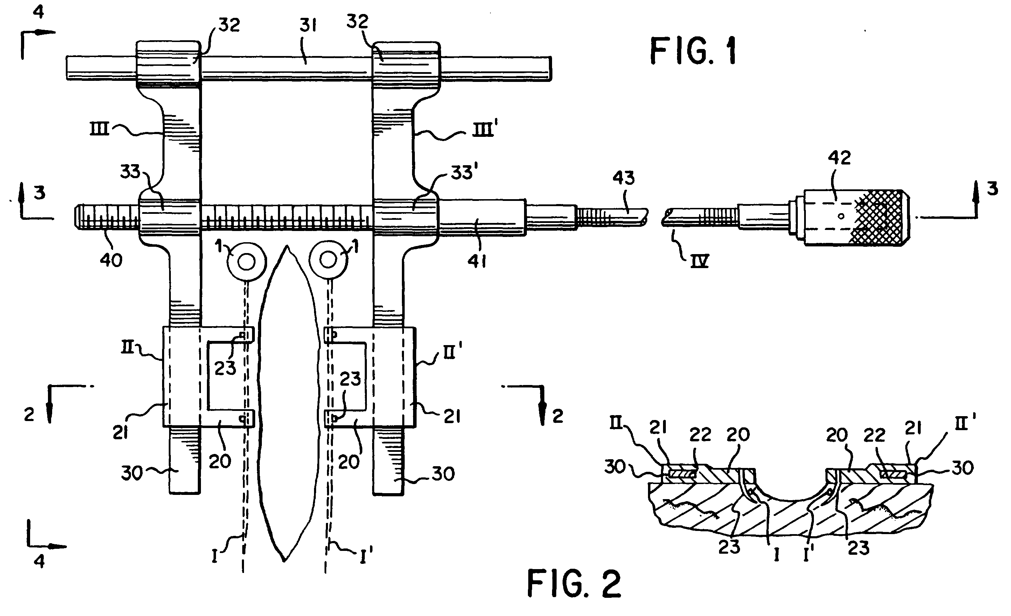

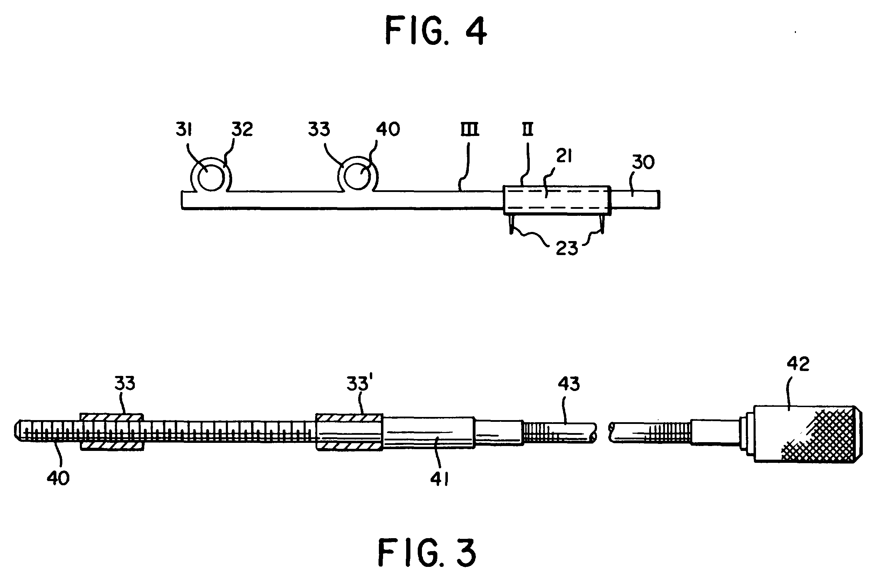

With reference to Figures 1 through 4 of the drawings the skin stretching apparatus comprises two long pins I and I' each provided with a head 1 which are inserted under the skin along the wound margins as shown in Figures 1 and 2, and two retaining members II and II' each engaging one of the two pins. Each retaining member is in U-shape and comprises two parallel, spaced-apart legs 20 connected by a flange 21 which is longitudinally perforated by a bore of rectangular cross section (22). Forwardly extending hooks 23 are connected to the underside of the members one each at the end of each leg 20 which are shown to have pierced the skin and to have engaged the pins from the side remote from the wound. The retaining members are held in position by two contractor arms III and III' and are slidingly mounted on the bar-shaped ends 30 by means of their rectangular bores 22. The contractor arms are held in parallel alignment by a cylindrical bar 31 which extends through horizontal bores in lugs 32 at the other ends of the arms. A screw IV is adapted to pull the two arms together and extends through a screw-threaded bore in a lug 33 on the top surface of the arms III and through a smooth bore in a lug 33' on the top surface of the arm III'. The screw IV comprises a screw-threaded end 40, a collar 41 abutting to the lug 33' of arm III' and a handle or knob 42 attached to the screw IV by means of a flexible tube or helical spring 43. By rotating the handle 42 the screw-threaded end in engagement with the thread in the lug 33 pulls the arms III and III' to each other whereby the retaining members II and II' contract the wound margins by means of the hooks 23 and the pins I and I'. As described in the previous patent, the operation is performed in several stages to allow the skin to stretch in a gradual manner during intervals between the stretching stages. After the wound margins have been contacted the wound is sutured in a manner known to the art, whereby it is evident that the legs 20 do not interfere with the operation, being remote from the margins.

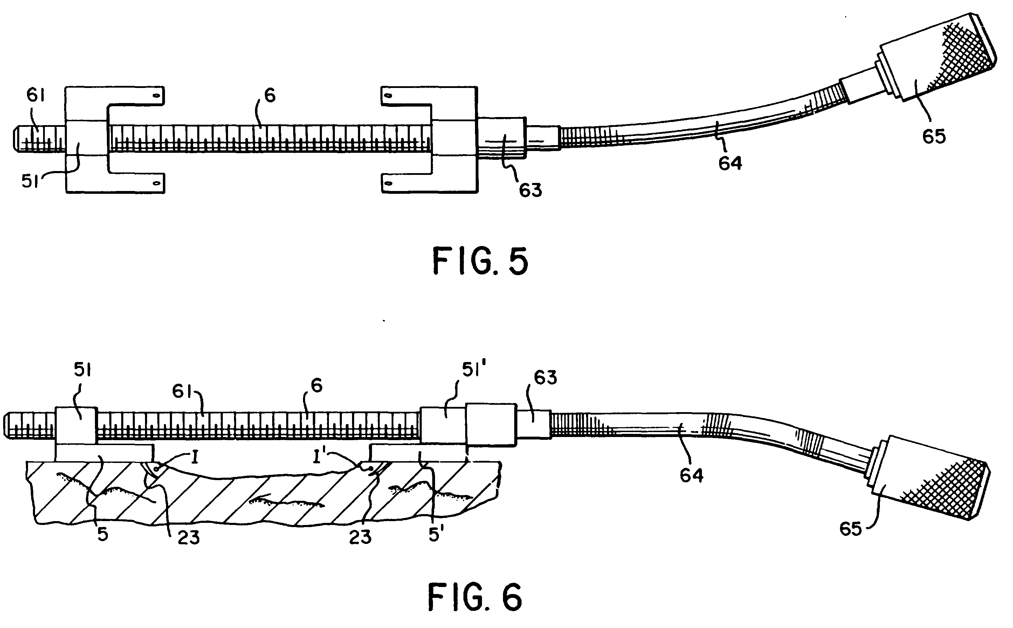

A second embodiment of the apparatus is illustrated in Figures 5 and 6, wherein two retaining members 5 and 5' are contracted by a screw 6. The members which are essentially identical with those shown in Figures 1 and 2, are provided with perforated lugs 51 and 51' on their respective top surfaces, lug 51 being perforated by a screw-threaded bore and lug 51' by a smooth bore.

The screw 6 comprises a screw-threaded end 61 cooperating with the screw thread in lug 51 of member 5, a smooth portion 62 rotating in lug 51', a collar 63 abutting lug 51' and a flexible operating portion 64 terminating in a knob or handle 65. The skin contracting operation is similar to that described with reference to the former embodiment, however it becomes evident that the screw 6 somewhat obstructs the suturing operation.

## Claims

1. Apparatus for the closing of skin defects by gradual stretching of skin comprising two pins (I I') serving for insertion underneath the skin close to the margins of a skin defect to be closed, two U-shaped retaining members (11,11') each having a top surface, and a bottom surface to be positioned close to the skin, each member being provided with skin piercing elements (23) at the end of each leg (20) of said "U" protruding out of its bottom surface, said elements serving for insertion into the skin behind each of said pins and to engage said pins, with said elements on said two members positionable in opposite alignment, contracting means 40, (111,111')connecting said two retaining members and adapted to approximate said retaining members and said pins by screw means (43) until final closure of said skin defect.

2. Apparatus as defined in claim 1 wherein said contracting means comprise two contractor arms (30) in parallel alignment, each said arm carrying one of said retaining members in sliding engagement, said contractor arms being connected by screw means for mutual approximation by manual rotation of said screw means.

3. Apparatus as defined in claim 2 wherein each said contractor arm includes a straight and smooth bar (30) at its one end, and wherein each said retaining member comprises two legs connected by a flange (21) remote from said skin margin, said flange being longitudinally perforated by a bore (22) cooperating with said bar of said contractor arm and mounted on said bar in sliding engagement.

4. Apparatus as defined in claim 3 wherein each of said contractor arms is provided with a bore perpendicular to said straight bar, and wherein a screw (IV) extends through said bores in said two arms connecting said two arms, and wherein one of said arms is provided with a smooth bore and the other with a screw-threaded bore cooperating with the screw thread of said screw.

5. Apparatus as defined in claim 4 where said screw connecting said two contractor arms includes a screw-threaded portion engaging said screw-threaded bore, with a collar abutting the smooth bore on the side remote from said skin defect and with a handle or knob (42) for manually rotating said screw.

6. Apparatus as defined in claim 4 wherein said two contractor arms are provided with coaxial bores at the ends remote from said straight bars and are connected by a cylindrical bar (31) extending through said two bores in sliding engagement.

7. Apparatus as defined in claim 6 wherein each contractor arm has a bottom surface to be positioned close to the skin and a top surface remote from the skin and wherein said bores are provided in lugs protruding out of the top surface of said arms.

8. Apparatus as defined in claim 1 wherein each of said two U-shaped retaining members comprises two parallel legs connected by a flange perforated by a bore (51) in parallel alignment with said legs, and wherein said retaining members are connected by a screw means (64) extending through said bores and are adapted to be approximated by rotation of said screw means in screw-thread provided in at least one of said bores.

9. Apparatus according to claim 8 wherein one of said U-shaped retaining member bores is a threaded bore and another of said U-shaped retaining member bores is a smooth bore, said contracting mechanism comprising at least a screw extending through said U-shaped retaining member bores.

10. Apparatus according to any of claims 3 to 9 wherein each of said retaining members is configured so that said retaining member legs are in parallel alignment.

## Patentansprüche

1. Vorrichtung zum Verschließen von Hautwunden durch allmähliches Spannen der Haut, die zwei Nadeln (I, I'), die zur Einführung unter die Haut nahe der Ränder einer zu verschließenden Hautwunde dienen, zwei U-förmige Halteelemente (II, II'), die jeweils eine Oberseite und eine Unterseite aufweisen, die nahe der Haut positioniert ist wobei jedes Element mit Hautdurchstichteilen (23) am Ende jedes Schenkels (20) des besagten 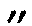 U" ausgestattet ist die aus ihrer Unterseite herausragen, besagte Teile zur Einführung in die Haut hinter jeder der besagten Nadeln dienen und um besagte Nadeln mit besagten Teilen an den besagten beiden Elementen zu greifen, die in entgegengesetzter Stellung positionierbar sind, und Verengungsmittel(40, III, III') umfaßt, die besagte zwei Halteelemente verbinden und angepaßt sind, um besagte Halteelemente und besagte Nadeln durch eine Schraubeinrichtung (43) bis zum endgültigen Verschließen der besagten Hautwunde anzunähern.

2. Vorrichtung nach Anspruch 1, dadurch gekennzeichnet daß das besagte Verengungsmittel zwei Verengerarme (30) in paralleler Stellung umfaßt, wobei jeder Arm eines der besagten Halteelemente in gleitendem Eingriff trägt, besagte Verengerarme durch eine Schraubeinrichtung zur wechselseitigen Annäherung durch manuelle Drehung der besagten Schraubeinrichtung verbunden sind.

3. Vorrichtung nach Anspruch 2, dadurch gekennzeichnet daß jeder Verengerarm eine gerade und glatte Schiene(30) an seinem einen Ende einschließt und daß jedes Halteelement zwei Schenkel umfaßt, die durch einen von besagtem Hautrand entfernten Flansch (21) verbunden sind, wobei besagter Flansch in Längsrichtung durch eine Bohrung (22) durchbohrt ist, die mit besagter Schiene des besagten Verengerarms zusammenwirkt und auf besagter Schiene in gleitendem Eingriff montiert ist.

4. Vorrichtung nach Anspruch 3, dadurch gekennzeichnet daß jeder der Verengerarme mit einer zu der geraden Schiene senkrechten Bohrung versehen ist, daß sich eine Schraube (IV) durch die Bohrungen in den beiden Armen erstreckt, die beide Arme verbindet, und daß einer der Arme mit einer glatten Bohrung und der andere mit einer Bohrung mit Gewindegang, die mit dem Gewindegang der Schraube zusammenwirken, ausgestattet ist.

5. Vorrichtung nach Anspruch 4, dadurch gekennzeichnet, daß die Schraube, die die beiden Verengerarme verbindet, einen Gewindegangabschnitt, der in die Bohrung mit Gewindegang greift, mit einer Hälse, die an die glatte Bohrung an der von der Hautwunde entfernten Seite angrenzt und mit einem Griff oder Bedienungsknopf (42) zum manuellen Drehen der Schraube einschließt.

6. Vorrichtung nach Anspruch 4, dadurch gekennzeichnet daß die beiden Verengerarme mit koaxialen Bohrungen an den von den geraden Schienen entfernten Enden ausgestattet sind und durch eine Zylinderschiene (31) verbunden sind, die sich durch die beiden Bohrungen in gleitendem Eingriff erstreckt.

7. Vorrichtung nach Anspruch 6, dadurch gekennzeichnet daß jeder Verengerarm eine Unterseite, die nahe der Haut positioniert ist und eine von der Haut entfernte Oberseite hat und daß die Bohrungen in Ansätzen vorliegen, die aus der Oberseite der Arme herausragen.

8. Vorrichtung nach Anspruch 1, dadurch gekennzeichnet daß jeder der beiden U-förmigen Halteelemente zwei parallele Schenkel umfaßt, die durch einen Flansch verbunden sind, der durch eine Bohrung (51) in paralleler Stellung zu den Schenkeln durchbohrt ist und daß die Halteelemente durch eine Schraubeinrichtung (64) verbunden sind, die sich durch besagte Bohrungen erstrecken und angepaßt sind, sich durch Drehung der Schraubeinrichtung in dem Schraubengang, der in wenigstens einer der Bohrungen besteht, anzunähern.

9. Vorrichtung nach Anspruch 8, dadurch gekennzeichnet daß eine der Bohrungen in den U-förmigen Halteelementen eine Bohrung mit Gewinde und die andere Bohrung in den U-förmigen Halteelementen eine glatte Bohrung ist wobei besagter Verengungsmechanismus wenigstens eine Schraube umfaßt, die sich durch die Bohrungen der U-förmigen Halteelemente erstreckt.

10. Vorrichtung nach einem der Ansprüche 3 bis 9, dadurch gekennzeichnet, daß jedes der Halteelemente so aufgebaut ist, daß die Schenkel der Halteelemente in paralleler Stellung sind.

## Revendications

1. Dispositif destiné à la fermeture de défauts de la peau par étirement graduel de la peau, comportant deux aiguilles (I, I') destinées à être insérées en dessous de la peau à proximité des bords d'un défaut de peau à fermer, deux organes de retenue en forme de U (11, 11') ayant chacun une surface supérieure et une surface inférieure, destinés à être positionnés à proximité de la peau, chaque organe étant muni d'éléments de perçage de peau (23) à l'extrémité de chaque jambe (20) dudit "U" faisant saillie à l'extérieur de sa surface inférieure, lesdits éléments étant destinés à être insérés dans la peau derrière chacune desdites aiguilles et à venir en contact avec lesdites aiguilles, lesdits éléments situés sur lesdits deux organes pouvant être positionnés en étant alignés en vis-à-vis, des moyens de contraction (111, 111') reliant lesdits deux organes de retenue et étant adaptés pour rapprocher lesdits organes de retenue et lesdites aiguilles par des moyens formant vis (43) jusqu'à la fermeture finale dudit défaut de peau.

2. Dispositif selon la revendication 1, dans lequel lesdits moyens de contraction comportent deux bras de contraction (30) alignés parallèlement, chaque dit bras supportant un desdits organes de retenue en contact coulissant, lesdits bras de dispositif de contraction étant reliés par des moyens formant vis destinés à leur rapprochement mutuel par mise en rotation manuelle desdits moyens formant vis.

3. Dispositif selon la revendication 2, dans lequel chaque dit bras de dispositif de contraction comporte une barre rectiligne et lisse (30) à sa première extrémité, et dans lequel chaque dit organe de retenue comporte deux jambes reliées par un rebord (21) éloigné dudit bord de peau, ledit rebord étant perforé longitudinalement par un alésage (22) coopérant avec ladite barre dudit bras de dispositif de contraction, et étant monté sur ladite barre en contact coulissant.

4. Dispositif selon la revendication 3, dans lequel chacun desdits bras de dispositif de contraction est muni d'un alésage perpendiculaire à ladite barre rectiligne, et dans lequel une vis (IV) s'étend à travers lesdits alésages desdits deux bras en reliant lesdits deux bras, et dans lequel un desdits bras est muni d'un alésage lisse et l'autre est muni d'un alésage taraudé coopérant avec le pas de vis de ladite vis.

5. Dispositif selon la revendication 4, dans lequel ladite vis reliant lesdits deux bras de dispositif de contraction comporte une partie filetée venant en prise avec ledit alésage taraudé, un collier venant en butée avec l'alésage lisse sur le côté éloigné par rapport audit défaut de peau, et une poignée ou bouton (42) pour mettre ladite vis manuellement en rotation.

6. Dispositif selon la revendication 4, dans lequel lesdits deux bras de dispositif de contraction sont munis d'alésages coaxiaux aux extrémités éloignées par rapport auxdites barres rectilignes et sont reliés par une barre cylindrique (31) s'étendant en contact coulissant à travers lesdits alésages.

7. Dispositif selon la revendication 6, dans lequel chaque bras de dispositif de contraction a une surface inférieure destinée à être positionnée à proximité de la peau et une surface supérieure éloignée de la peau et dans lequel lesdits alésages sont agencés dans des pattes faisant saillie à l'extérieur de la surface supérieure desdits bras.

8. Dispositif selon la revendication 1, dans lequel chacun desdits deux organes de retenue en forme de U comporte deux jambes parallèles reliées par un rebord perforé par un alésage (51) aligné parallèlement auxdits jambes, et dans lequel lesdits organes de retenue sont reliés par des moyens formant vis (64) s'étendant à travers lesdits alésages et sont adaptés pour être rapprochés par mise en rotation desdits moyens formant vis dans un pas de vis agencé dans au moins un desdits alésages.

9. Dispositif selon la revendication 8, dans lequel un premier des alésages d'organe de retenue en forme de U est un alésage taraudé et l'autre desdits alésages d'organe de retenue en forme de U est un alésage lisse, ledit mécanisme de contraction comportant au moins une vis s'étendant à travers lesdits alésages d'organe de retenue en forme de U.

10. Dispositif selon l'une quelconque des revendications 3 à 9, dans lequel chacun desdits organes de retenue est configuré de sorte que lesdites jambes d'organe de retenue sont parallèlement alignées.
